# FASCICULE DE BREVET EUROPEEN

(11) **EP 0 738 328 B1**
(45) Date de publication et mention de la délivrance du brevet: **07.12.2005**
(21) Numéro de dépôt: 95906377.7
(22) Date de dépôt: 09.01.1995
(51) Int. Cl.: C12N 15/87, A61K 47/48, A61K 48/00, A61K 9/127

(54) **COMPOSITION CONTENANT DES ACIDES NUCLEIQUES, PREPARATION ET UTILISATIONS**
NUKLEINSAEURE ENTHALTENDE ZUSAMMENSETZUNGEN, HERSTELLUNG UND VERWENDUNG
COMPOSITION CONTAINING NUCLEIC ACIDS, PREPARATION AND USES

(30) Priorité: 10.01.1994 FR 9400159
(43) Date de publication de la demande: 23.10.1996
(73) Titulaire: Aventis Pharma S.A., 92160 Antony (FR)
(72) Inventeur: BEHR, Jean-Paul, F-67100 Strasbourg (FR); DEMENEIX, Barbara, F-75013 Paris (FR); REMY, Jean-Serge, F-67100 Strasbourg (FR); SCHERMAN, Daniel, F-75645 Paris Cédex 13 (FR); SCHWARTZ, Bertrand, F-94700 Maisons-Alfort (FR)
(74) Mandataire: Peaucelle, Chantal
(86) Numéro de dépôt international: PCT/FR1995/000022
(87) Numéro de publication internationale: WO 1995/018863

(56) Documents cités:
- WO-A-93/24640
- WO-A-94/00569
- PROCEEDINGS OF THE NATIONAL ACADEMY OF SCIENCES OF USA, vol.86, Septembre 1989, WASHINGTON US pages 6982 - 6986 J.-P. BEHR ET AL 'Efficient gene transfer into mammalian primary endocrine cells with lipopolyamine-coated DNA'
- METHODS IN ENZYMOLOGY, vol.217, 1993 pages 599 - 618 J.P. LOEFFLER ET AL 'Gene transfer into primary and established mammalian cell lines with lipopolyamine-coated DNA'
- JOURNAL OF LIPOSOME RESEARCH, vol.3, no.1, 1993, NEW YORK US pages 17 - 30 XIANG GAO ET AL 'Cationic liposomes and polymers for gene transfer'
- THE INTERNATIONAL JOURNAL OF DEVELOPMENTAL BIOLOGY, vol.35, no.4, Décembre 1991 pages 481 - 484 B.A. DEMENEIX ET AL 'Gene transfer into intact vertebrate embryos' cité dans la demande
- HUMAN GENE THERAPY, vol.3, 1992 pages 267 - 275 M.J. STEWART ET AL 'Gene transfer in vivo with DNA-liposome complexes : Safety and acute toxicity in mice'
- PROCEEDINGS OF THE NATIONAL ACADEMY OF SCIENCES OF USA, vol.86, Août 1989, WASHINGTON US pages 6077 - 6081 R.W. MALONE ET AL 'Cationic liposome-mediated RNA transfection'

## Description

La présente invention concerne des compositions à base d'acides nucléiques, leur préparation et leur utilisation. Plus particulièrement, elle concerne des compositions comprenant au moins un acide nucléique et une lipopolyamine et leur utilisation en thérapie génique, notamment pour le transfert d'acides nucléiques.

La thérapie génique consiste à corriger une déficience ou une anomalie (mutation, expression aberrante, etc) ou à assurer l'expression d'une protéine d'intérêt thérapeutique par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. Différentes techniques ont été décrites pour le transfert de cette information génétique, parmi lesquelles des techniques diverses de transfection impliquant des complexes d'ADN et de DEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), d'ADN et de polylysine, l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physicochimiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

Toutefois, les techniques développées jusqu'à présent ne permettent pas de résoudre de manière satisfaisante les difficultés liées au transfert de gènes dans les cellules et/ou l'organisme. En particulier, les problèmes liés à la pénétration de l'acide nucléique dans les cellules ne sont pas entièrement résolus. En effet, la nature polyanionique des acides nucléiques prévient leur passage à travers les membranes cellulaires. S'il a été montré que les acides nucléiques nus sont capables de traverser la membrane plasmique de certains types cellulaires in vivo (voir notamment la demande n° WO90/11092), l'efficacité de transfection reste assez faible. De plus, les acides nucléique nus ont une demi-vie plasmatique courte, en raison de leur dégradation par les enzymes et de leur élimination par les voies urinaires. Par ailleurs, si les virus recombinants permettent d'améliorer l'efficacité de transfert des acides nucléiques, leur emploi présente certains risques tels que la pathogénicité, la transmission, la réplication, la recombinaison, la transformation, l'immunogénicité, etc.

La présente invention apporte une solution avantageuse à ces differents problèmes. La demanderesse a en effet montré que certaines compositions comprenant un acide nucléique et une lipopolyamine dans laquelle la région polyamine est la spermine peuvent permettre le transfert in vivo dudit acide nucléique dans une cellule et/ou un organe avec une grande efficacité et sans toxicité. Les compositions de l'invention permettent également d'éviter les inconvénients liés à l'emploi de vecteurs viraux (dangers potentiels, taille limitée du gène transféré, prix élevé, etc).

L'utilisation de certaines lipopolyamines pour transfecter in vitro des cultures cellulaires a déjà été décrite dans l'art antérieur. Ainsi, la demande EP 394 111 décrit l'utilisation de certaines lipopolyamines pour la transfection de lignées cellulaires in vitro. De même, l'article de Demeneix et al (Int. J. Dev. Biol. 35 (1991) 481) décrit l'utilisation d'une lipopolyamine (la dioctadécylamidoglycyl spermine, DOGS) pour la transfection d'acides nucléiques in ovo. Selon ces documents, les lipopolyamines doivent être utilisées dans des conditions telles que le rapport charges positives de la lipopolyamine / charges négatives de l'acide nucléique soit compris entre 2 et 5, et de préférence égal à 3 ou 4. Cependant, comme le montrent les exemples 8 et 9 de la présente demande, de manière surprenante, aucune des conditions décrites dans ces documents ne permet la transfection d'acides nucléiques in vivo. En raison d'interaction avec des macromolécules anioniques ou avec la matrice extracellulaire des tissus, les particules formées dans ces conditions sont en effet incapables de diffuser hors du site d'application, et donc de transférer tout acide nucléique in vivo. De plus, les conditions de préparation décrites dans ces documents ne sont pas applicables à la réalisation de compositions pharmaceutiques contenant des quantités importantes d'acides nucléiques. La demanderesse a maintenant montré que, dans certaines conditions, les lipopolyamines peuvent être utilisées pour la transfection in vivo d'acides nucléiques. Plus particulièrement, la demanderesse a trouvé que, de manière surprenante, des compositions comprenant un acide nucléique et une lipopolyamine dans des conditions telles que le rapport charges positives de la lipopolyamine / charges négatives de l'acide nucléique soit inférieur ou égal à 2 permettent la transfection dudit acide nucléique in vivo avec une grande efficacité.

De plus, la demanderesse a mis au point certaines conditions permettant la préparation de ces compositions pharmaceutiques incorporant des quantités importantes d'acide nucléique. Les compositions pharmaceutiques de l'invention constituent ainsi des outils particulièrement avantageux pour l'administration et le transfert d'acides nucléiques in vivo.

Les articles par Behr J.P. et al., Proceedings of the National Academy of Sciences of USA, vol. 86, 1989, pp. 6982-6986 et Loeffler J.P. et al., Methods in Enzymology, vol. 217, 1993, pp 599-618 divulguent des compositions de trsnfection in vitro comprenant un acide nucléique et une lipopolyamine du type DOGS ou DPPES. Cependant, ces compositions ne sont pas utilisées comme médicament et ne comportent pas de lipide neutre DOPE.

L'article par Xiang Gao et al., Journal of Liposome Research, vol. 3, n°1, 1993, pp 17-30 décrit des compositions de transfection d'un acide nucléique dont certaines comportent une lipopolyamine (polylysine) et le lipide neutre DOPE. L'importance des valeurs du rapport de charge n'est cependant pas indiquée. De plus, les lipopolyamines utilisées dans les compositions selon l'invention ne sont pas des polylysines mais des lipopolyamines à tête spermine.

Un premier objet de l'invention réside donc dans une composition comprenant au moins un acide nucléique et une lipopolyamine dans laquelle la région polyamine est la spermine, et dont le rapport charges positives de la lipopolyamine sur charges négatives de l'acide nucléique est inférieur ou égal à 2, pour une utilisation comme médicament.

Au sens de la présente invention, le terme lipopolyamine désigne toute molécule amphiphile comprenant au moins une région hydrophile polyamine et une région lipophile. La région polyamine des lipopolyamines, chargée cationiquement, est capable de s'associer de manière réversible avec l'acide nucléique, chargé négativement. Cette interaction compacte fortement l'acide nucléique. La région lipophile rend cette interaction ionique insensible au milieu externe, en recouvrant la particule nucléolipidique formée d'une pellicule lipidique.

Avantageusement, la région polyamine des lipopolyamines utilisées dans le cadre de l'invention répond à la formule générale 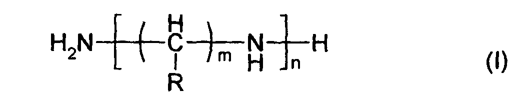 dans laquelle n et m sont des nombres entiers choisis de manière à former un groupement spermine,
- R représente un atome d'hydrogène ou un radical de formule générale : 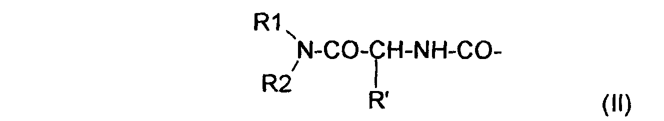 dans lequel R₁ et R₂, identiques ou différents, représentent chacun un radical aliphatique saturé CₚH₂ₚ₊₂ ou insaturé CₚH₂ₚ ou CₚH₂ₚ₋₂, p étant un nombre entier compris entre 12 et 22 inclusivement, et R' représente un atome d'hydrogène ou un radical alkyle contenant 1 à 4 atomes de carbone éventuellement substitué par un radical phényle, ou un radical de formule générale: 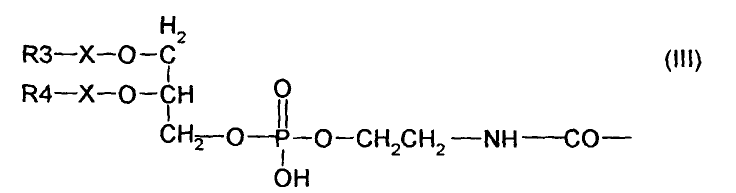 dans lequel X représente un groupement méthylène (-CH₂-) ou un groupement carbonyle (-CO-), et R₃ et R₄, identiques ou différents, représentent chacun un radical aliphatique saturé C_{p'}H_{2p'+2} ou insaturé C_{p'}H_{2p'} ou C_{p'}H_{2p'-2}, p' étant un nombre entier compris entre 11 et 21 inclusivement, étant entendu que :
   - quelles que soient les valeurs de m et n, un seul des symboles R représente un radical de formule générale (II) ou (III).

La région lipophile peut être une chaîne hydrocarbonée, saturée ou non, du cholestérol, un lipide naturel ou un lipide synthétique capables de former des phases lamellaires ou héxagonales.

Avantageusement, on utilise dans le cadre de la présente invention des lipopolyamines telles que définies dans la demande de brevet EP 394 111. Cette demande décrit également un procédé utilisable pour la préparation de ces lipopolyamines.

De manière particulièrement avantageuse, on utilise dans le cadre de l'invention la dioctadécylamidoglycyl spermine (DOGS) ou la 5-carboxyspermylamide de la palmitoylphosphatidylethanolamine (DPPES).

Pour obtenir un effet optimum des compositions de l'invention, les proportions respectives de la polyamine et de l'acide nucléique sont de préférence déterminées de sorte que le rapport R charges positives de la lipopolyamine / charges négatives de l'acide nucléique soit compris entre 0,1 et 1,9; plus préférentiellement entre 0,5 et 1,5.

Dans les compositions de la présente invention, l'acide nucléique peut être aussi bien un acide désoxyribonucléique qu'un acide ribonucléique. Il peut s'agir de séquences d'origine naturelle ou artificielle, et notamment d'ADN génomique, d'ADNc, d'ARNm, d'ARNt, d'ARNr, de séquences hybrides ou de séquences synthétiques ou semi-synthétiques. Ces acides nucléiques peuvent être d'origine humaine, animale, végétale, bactérienne, virale, etc. Ils peuvent être obtenus par toute technique connue de l'homme du métier, et notamment par criblage de banques, par synthèse chimique, ou encore par des méthodes mixtes incluant la modification chimique ou enzymatiqe de séquences obtenues par criblage de banques. Ils peuvent par ailleurs être incorporés dans des vecteurs, tels que des vecteurs plasmidiques.

Concernant plus particulièrement les acides désoxyribonucléiques, ils peuvent être simple ou double brin. Ces acides désoxyribonucléiques peuvent porter des gènes thérapeutiques, des séquences régulatrices de la transcription ou de la réplication, des séquences antisens, des régions de liaison à d'autres composants cellulaires, etc.
Au sens de l'invention, on entend par gène thérapeutique notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.
Parmi les produits thérapeutiques au sens de la présente invention, on peut citer plus particulièrement les enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, HARP/pléiotrophine, etc; les apolipoprotéines : ApoAI, ApoAIV, ApoE, etc (FR 93 05125), la dystrophine ou une minidystrophine (FR 9111947), la protéine CFTR associée à la mucoviscidose, les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, les gènes intervenant dans la réparation de l'ADN, les gènes suicides (thymidine kinase, cytosine déaminase), etc.

Le gène thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les antisens comprennent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, ou encore spécifiques de tumeurs (EP 259 212).

Préférentiellement, l'acide nucléique comprend également des séquences permettant l'expression du gène thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc. En outre, ces séquences d'expression peuvent être modifiées par addition de séquences d'activation, de régulation, etc.
Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

Dans un autre mode de mise en oeuvre, la présente invention concerne des compositions comprenant un acide nucléique, une lipopolyamine dans laquelle la région lipopolyamine est la spermine et un adjuvant capable de s'associer au complexe lipopolyamine/acide nucléique et d'améliorer le pouvoir transfectant, le rapport charges positives de la lipopolyamine sur charges négatives de l'acide nucléique étant inférieur ou égal à 2. La demanderesse a en effet montré que le pouvoir transfectant des lipopolyamines peut être de manière inattendue augmenté en présence de certains adjuvants (lipides ou protéines par exemple), capables de s'associer au complexe lipopolyamine/acide nucléique. Comme le montrent les exemples 4 à 7 de la présente demande, cette amélioration se manifeste aussi bien in vitro que in vivo.

Plus préférentiellement, les compositions de l'invention comprennent, comme adjuvant, un ou plusieurs lipides neutres. De telles compositions sont particulièrement avantageuses, notamment lorsque le rapport R est faible. La demanderesse a en effet montré que l'addition d'un lipide neutre permet d'améliorer la formation des particules nucléolipidiques et, de manière surprenante, de favoriser la pénétration de la particule dans la cellule en déstabilisant sa membrane.
Plus préférentiellement, les lipides neutres utilisés dans le cadre de la présente invention sont des lipides à 2 chaines grasses.
De manière particulièrement avantageuse, on utilise des lipides naturels ou synthétiques, zwitterioniques ou dépourvus de charge ionique dans les conditions physiologique. Il peuvent être choisis plus particulièrement parmi la dioleoylphosphatidyléthanolamine (DOPE), le oléoyl-palmitoylphosphatidyléthanolamine (POPE), le di-stéaroyl, -palmitoyl, -mirystoyl phosphatidyléthanolamine ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines)ou encore les asialogangliosides (tels que notamment les asialoGM1 et GM2).
Ces différents lipides peuvent être obtenus soit par synthèse, soit par extraction à partir d'organes (exemple : le cerveau) ou d'oeufs, par des techniques classiques bien connues de l'homme du métier. En particulier, l'extraction des lipides naturels peut être réalisée au moyen de solvants organiques (voir également Lehninger, Biochemistry).

Préférentiellement, les compositions de l'invention comprennent de 0,1 à 20 équivalents d'adjuvant pour 1 équivalent de lipopolyamine, et, plus préférentiellement, de 1 à 5.

Les compositions selon l'invention peuvent être formulées en vue d'administrations par voie topique, cutanée, orale, rectale, vaginale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée.

La présente invention fournit ainsi des compositions utilisables comme médicament, particulièrement avantageuse pour le traitement de maladies, comprenant l'administration in vivo d'un acide nucléique apte à corriger ladite maladie associé à une lipopolyamine dans les conditions définies ci-avant. Plus particulièrement, les compositions selon l'invention sont applicables aux traitement de maladies résultant d'une déficience en un produit protéique ou nucléique et l'acide nucléique administré code pour ledit produit protéique ou contient ledit produit nucléique.

La présente invention sera plus complètement décrite à l'aide des exemples qui suivent, qui doivent être considérés comme illustratifs et non limitatifs.

### EXEMPLE 1 - Plasmides utilisés pour le transfert de gènes in vivo

Trois types de constructions ont été utilisées pour mettre en évidence l'activité des compositions de l'invention : Des plasmides comportant le gène codant pour la luciférase (Luc), des plasmides comportant le gène codant pour la β-galactosidase (gène LacZ) et des plasmides comportant le gène codant pour la chloramphénicol acétyl transférase (CAT).

### 1.1. Plasmides comportant le gène Luc

Le plasmide pCMV-luc comporte le promoteur du Cytomégalovirus (CMV), extrait du plasmide vecteur pcDNA3 (Invitrogen) par coupure avec les enzymes de restriction Mlu I et HindIII, situé en amont du gène codant pour la luciférase, inséré aux sites MluI et HindIII dans le vecteur pGL basic Vector (Promega).

### 1.2. Plasmides comportant le gène LacZ

Le plasmide pCMV-βGal (Clontech) comporte le promoteur CMV situé en amont du gène LacZ codant pour la β-galactosidase d'Escherichia coli. Le vecteur pSV-nls LacZ (pAOnlsLacZ) comporte le même promoteur, une séquence de localisation nucléaire (issue du virus SV40) localisée en phase et en amont du gène LacZ. Cette construction permet l'expression de la protéine de fusion nls-β-galactosidase dans le noyau des cellules (CfDe Luze et al., PNAS 90 (1993) 7322).

### 1.3. Plasmides comportant le gène CAT

Les plasmides ayant pour gène rapporteur le gène codant pour la chloramphénicol acétyl transférase (CAT) sous contrôle des promoteurs RSV (pRSV-CAT) et SV40 (pSV40-CAT) ont été publiés (Boutiller et al., Prog. Neuro-PhychoPharmacol. et Biol. Psychiat. 16 (1992) 959; de Luze et al. PNAS 90 (1993) 7322).

### EXEMPLE 2 - Transfert d'acide nucléique in vivo dans le cerveau de souris nouveau-nées en utilisant une lipopolyamine dans un rapport R = 0,8.

Cet exemple décrit le transfert du plasmide pCMV-luc in vivo sur le cerveau de souris nouveau-nées.
30 µg de plasmide pCMV-luc (exemple 1.1.) ont été dilués dans 30 µl NaCl 150 mM stérile (concentration de 1 µg/µl). Ensuite, 0.6 µl de dioctadécylamidoglycyl spermine (DOGS) 40 mM, préparé dans de l'éthanol 100 %, ont été ajoutés.

Le mélange a été vortexé rapidement et utilisé pour des injections intracérébrales chez des souris nouveau-nées. Pour cela, les souris ont été anesthésiées par le froid (placées sur une feuille d'aluminium en contact avec de la glace), puis 2 µl de mélange (2 µg d'acide nucléique) ont été injectés par souris. Les injections ont été réalisées dans le cortex, à l'aide d'un micromanipulateur et une microseringue reliées à une microélectrode.
Les cerveaux ont été prélevés 48 heures plus tard, homogénéisés, centrifugés et le surnageant utilisé pour le dosage de la luciférase. Pour cela, le surnageant a été incubé en présence d'un tampon comprenant de la luciférine, du coenzyme A et de l'ATP, et la lumière émise (généralement pendant 10 secondes) a été mesurée avec un luminomètre (Wood K. (1990) Promega Notes, 28).
Les résultats obtenus montrent une activité en unités lumière relative normalisée de 425 RLU/cerveau (moyenne de 10 animaux) (RLU = "relative light unit") lorsque le transfert est réalisé au moyen de la composition de l'invention avec R=0,8, contre une activité normalisée de 100 RLU/cerveau lorsque le transfert est réalisé au moyen du plasmide seul (moyenne de 10 animaux).

### EXEMPLE 3 - Comparaison du transfert d'acide nucléique in vivo dans le cerveau de souris nouveau-nées en utilisant une lipopolyamine dans un rapport R = 1,5 et 0,8.

### 3.1. Transfert du plasmide pCMV-luc

Pour le rapport R=0,8, les conditions sont celles de l'exemple 2. Pour le rapport R=1,5; 30 µg de plasmide pCMV-luc (exemple 1.1.) ont été dilués dans 30 µl NaCl 150 mM stérile (concentration de 1 µg/µl). Ensuite, 1,13 µl de dioctadécylamidoglycyl spermine (DOGS) 40 mM, préparé dans de l'éthanol 100 %, ont été ajoutés.

Le mélange a été vortexé rapidement et utilisé pour des injections intracérébrales chez des souris nouveau-nées. Pour cela, les souris ont été anesthésiées par le froid (placées sur une feuille d'aluminium en contact avec de la glace), puis 2 µl de mélange (2 µg d'acide nucléique) ont été injectés par souris. Les injections ont été réalisées dans le cortex, à l'aide d'un micromanipulateur et une microseringue reliées à une microélectrode.
Les cerveaux ont été prélevés 48 heures plus tard, homogénéisés, centrifugés et le surnageant utilisé pour le dosage de la luciférase selon le protocole décrit dans l'exemple 2. Les résultats obtenus montrent une activité normalisée de 89 RLU/cerveau (moyenne de 12 animaux) lorsque le transfert est réalisé au moyen de la composition de l'invention avec un rapport R=1,5, contre une activité normalisée de 100 RLU/cerveau lorsque le transfert est réalisé au moyen de la composition de l'invention avec un rapport R=0,8 (moyenne de 11 animaux).

### 3.2. Transfert du plasmide pSV-nls LacZ

La même procédure que dans l'exemple 3.1. ci-dessus a été employée pour transfecter le plasmide pSV-nls LacZ comportant le gène LacZ codant pour la β-galactosidase sous contrôle d'un promoteur SV40 (simian virus 40). Cette construction (exemple 1.2.) code aussi pour un peptide de signalisation à localisation nucléaire. L'enzyme β-galactosidase est ainsi transportée vers le noyau et la réaction enzymatique produite par l'acide nucléique est limitée à cette région subcellulaire. Les injections ont été réalisées de la même manière que pour le plasmide pCMV-luc, et les cerveaux ont aussi été prélevés 48 heures post-transfection, fixés dans le paraformaldéhyde (2 %) pendant 24 heures puis traités pour la réaction β-galactosidase. Les cerveaux ont ensuite été examinés pour les sites d'expression, les zones positives coupées au cryostat (15 µm), montées sur lames et photographiées. Dans 2 séries d'expériences indépendantes, trois animaux sur 10 montrent, au niveau des régions situées autour de la zone d'injection, des groupes de cellules dont le noyau présente une coloration bleue très prononcée, caractéristique de l'activité β-galactosidase.

### EXEMPLE 4 - Transfert d'acides nucléiques in vitro : optimisation du rapport lipopolyamine/adjuvant.

Cet exemple décrit le transfert d'acides nucléiques in vitro (sur cultures cellulaires) au moyen d'une composition selon l'invention comprenant l'acide nucléique, une lipopolyamine et un adjuvant (lipide neutre) dans différentes conditions.

10⁵ cellules des lignées fibroblastes 3T3 et hépatome humain HepG2 ont été incubées respectivement en présence de 1 et 2µg des plasmides pCMV-βGal ou pCMV-Luc dans différentes conditions :
- en présence de DOGS dans des rapports de charges R= 1 et 1,5
- en l'absence ou en présence de 1, 2, 5 ou 10 équivalents d'un adjuvant (DOPE).
Le pouvoir transfectant a ensuite été déterminé dans les conditions décrites à l'exemple 2 pour la luciférase, ou par mesure des pourcentages de cellules présentant un coloration bleue prononcée pour l'activité LacZ. Les résultats obtenus sont présentés dans les tableaux ci-dessous.

**Tableau 1 :**

| Activité Luciférase (en RLU/ 10 sec./mg Prot.) | | |
|---|---|---|
| **Conditions/Cellules** | **3T3** | **HepG2** |
| DOGS (R=1,5) | 1,7.10⁶ | 2.10² |
| DOGS (R=1,5) + 2 eq DOPE | 2.10⁷ | 8.10³ |

**Tableau 2 :**

| Activité LacZ | | |
|---|---|---|
| | **Fibroblastes 3T3** | |
| **Rapport de DOGS** | **1** | **1,5** |
| 0 eq DOPE | 0 | 5 |
| 1 eq DOPE | 8 | 30 |
| 2 eq DOPE | 48 | 69 |
| 5 eq DOPE | 15 | 25 |
| 10 eq DOPE | 5 | 10 |

### EXEMPLE 5 - Transfert d'acide nucléique in vivo dans le cerveau de souris nouveau-nées en utilisant une lipopolyamine dans un rapport R = 1 et un lipide neutre.

Dans cet exemple, une solution éthanolique de DOGS à 40 mM a été mélangée avec un volume égal d'une solution de dioleoylphosphatidylethanolamine (DOPE) à 80 mM, préparée dans un mélange chloroforme/éthanol (1/5). Ainsi pour un équivalent de DOGS la composition contient deux équivalents de DOPE.

30 µg de plasmide pCMV-luc (exemple 1.1.) ont été dilués dans 30 µl NaCl 150 mM stérile (concentration de 1 µg/µl). Ensuite, 1,5 µl du mélange DOGS/DOPE préparé ci-dessus ont été ajoutés.

La suite du protocole (injections, prélèvements, dosages) est identique à celle décrite à l'exemple 3.1. Les résultats obtenus montrent une activité normalisée de 241 RLU /cerveau (moyenne de 13 animaux) lorsque le transfert est réalisé en présence de l'adjuvant (lipide neutre), contre une activité normalisée de 100 RLU/cerveau lorsque le transfert est réalisé en présence du DOGS seul, dans le même rapport de charges R = 1 (moyenne de 13 animaux).

### EXEMPLE 6 - Transfert d'acide nucléique in vivo dans le cerveau de souris nouveau-nées en utilisant une lipopolyamine dans un rapport R = 1,25 et un lipide neutre.

Dans cet exemple, une solution éthanolique de DOGS à 40 mM a été mélangée avec un volume égal d'une solution de dioleoylphosphatidylethanolamine (DOPE) à 80 mM, préparée dans un mélange chloroforme/éthanol (1/5). Ainsi pour un équivalent de DOGS la composition contient deux équivalents de DOPE.
30 µg de plasmide pCMV-luc (exemple 1.1.) ont été dilués dans 30 µl NaCl 150 mM stérile (concentration de 1 µg/µl). Ensuite, 1,87 µl du mélange DOGS/DOPE préparé ci-dessus ont été ajoutés.

La suite du protocole (injections, prélèvements, dosages) est identique à celle décrite à l'exemple 3.1. Les résultats obtenus montrent une activité normalisée de 405 RLU/cerveau (moyenne de 8 animaux) lorsque le transfert est réalisé en présence de l'adjuvant, contre une activité normalisée de 100 RLU/cerveau (moyenne de 8 animaux) lorsque le transfert est réalisé en présence du DOGS seul, dans le même rapport de charges R = 1,25.

### EXEMPLE 7 - Transfert d'acide nucléique in vivo dans le cerveau de souris nouveau-nées en utilisant une lipopolyamine dans un rapport R = 1,5 et un lipide neutre.

Dans cet exemple, une solution éthanolique de DOGS à 40 mM a été mélangée avec un volume égal d'une solution de dioleoylphosphatidylethanolamine (DOPE) à 80 mM, préparée dans un mélange chloroforme/éthanol (1/5). Ainsi pour un équivalent de DOGS la composition contient deux équivalents de DOPE.

30 µg de plasmide pCMV-luc (exemple 1.1.) ont été dilués dans 30 µl NaCl 150 mM stérile (concentration de 1 µg/µl). Ensuite, 2,26 µl du mélange DOGS/DOPE préparé ci-dessus ont été ajoutés.

La suite du protocole (injections, prélèvements, dosages) est identique à celle décrite à l'exemple 4.1. Les résultats obtenus montrent une activité normalisée de 165 RLU/cerveau (moyenne de 10 animaux) lorsque le transfert est réalisé au moyen d'une composition de l'invention contenant un adjuvant (lipide neutre), contre une activité normalisée de 100 RLU/cerveau (moyenne de 11 animaux) lorsque le transfert est réalisé au moyen du DOGS seul, dans le même rapport de charges (R=1,5).

### EXEMPLE 8 - Transfert d'acide nucléique in vivo chez la souris en utilisant une lipopolyamine dans un rapport R = 3.

Cet exemple décrit le transfert du plasmide pCMV-luc in vivo sur des souris adultes. Les expériences ont été réalisées sur des souris adultes anesthésiées au pentobarbital.

### 8.1. Transfert par voie veineuse

Pour chaque souris, 100 µg de plasmide pCMV-luc ont été dilués dans 100 µl de NaCl 150 mM. Ensuite, 7,5 µl de DOGS 40 mM ont été ajoutés à la solution. La veine jugulaire des souris a ensuite été exposée par dissection, et la solution ci-dessus injectée dans la jugulaire en direction du coeur. La peau a ensuite été refermée avec des agrafes. 48 heures après l'injection, les souris ont été sacrifées par dislocation cervicale, et le foie, les poumons, la rate, le cerveau, les reins et un échantillon de muscle squelettique ont été prélevés. Après homogénéisation et centrifugation, les surnageants ont été utilisés pour doser la luciférase. Cette expérience à été réalisée sur 4 souris. Aucune expression de la luciférase n'a été mise en évidence dans les différents tissus testés.

### 8.2. Transfert par voie intramusculaire

60 µg de plasmide pCMV-luc ont été dilués dans 300 µl de NaCl 150 mM. Ensuite, 4,5 µl de DOGS 40 mM ont été ajoutés à la solution. 50 µl du mélange préparé ci-avant (10 µg d'ADN) ont ensuite été injectés pour chaque souris, à travers la peau, dans le muscle antérieur tibilalis. 48 heures après l'injection, les souris ont été sacrifées par dislocation cervicale, et les muscles injectés ont été prélevés. Après homogénéisation et centrifugation, les surnageants ont été utilisés pour doser la luciférase. Cette expérience à été réalisée sur 6 souris. Aucune expression de la luciférase n'a été décelée.

### EXEMPLE 9 - Transfert d'acide nucléique in vivo chez la souris en utilisant une lipopolyamine dans un rapport R = 4.

Cet exemple décrit le transfert du plasmide pCMV-luc in vivo sur des souris adultes. Les expériences ont été réalisées sur des souris adultes anesthésiées au pentobarbital.

### 9.1. Transfert par voie veineuse

Pour chaque souris, 100 µg de plasmide pCMV-luc ont été dilués dans 100 µl de NaCl 150 mM. Ensuite, 10 µl de DOGS 40 mM ont été ajoutés à la solution. La veine jugulaire des souris a ensuite été exposée par dissection, et la solution ci-dessus injectée dans la jugulaire en direction du coeur. La peau a ensuite été refermée avec des agrafes. 48 heures après l'injection, les souris ont été sacrifées par dislocation cervicale, et le foie, les poumons, la rate, le cerveau, les reins et un échantillon de muscle squelettique ont été prélevés. Après homogénéisation et centrifugation, les surnageants ont été utilisés pour doser la luciférase. Cette expérience à été réalisée sur 4 souris. Aucune expression de la luciférase n'a été mise en évidence dans les différents tissus testés.

### 9.2. Transfert par voie intramusculaire

60 µg de plasmide pCMV-luc ont été dilués dans 300 µl de NaCl 150 mM. Ensuite, 6 µl de DOGS 40 mM ont été ajoutés à la solution. 50 µl du mélange préparé ci-avant (10 µg d'ADN) ont ensuite été injectés pour chaque souris, à travers la peau, dans le muscle antérieur tibilalis. 48 heures après l'injection, les souris ont été sacrifées par dislocation cervicale, et les muscles injectés ont été prélevés. Après homogénéisation et centrifugation, les surnageants ont été utilisés pour doser la luciférase. Cette expérience à été réalisée sur 6 souris. Aucune expression de la luciférase n'a été décelée.

## Revendications

1. Composition comprenant un acide nucléique et une lipopolyamine, dans laquelle la région polyamine est la spermine **caractérisée en ce que** le rapport charges positives de la lipopolyamine sur charges négatives de l'acide nucléique est inférieur ou égal à 2, pour une utilisation comme médicament.

2. Composition selon la revendication 1, **caractérisée en ce que** la lipopolyamine répond à la formule générale : 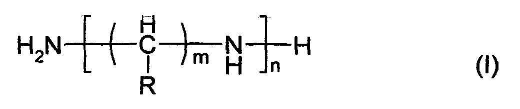
- n et m sont des nombres entiers choisis de manière à former un groupement spermine,
- R représente un atome d'hydrogène ou un radical de formule générale : 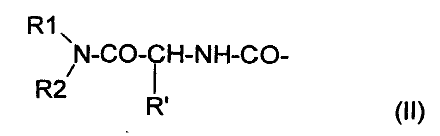 dans lequel R₁ et R₂, identiques ou différents, représentent chacun un radical aliphatique saturé CₚH₂ₚ₊₂ ou insaturé CₚH₂ₚ ou CₚH₂ₚ₋₂, p étant un nombre entier compris entre 12 et 22 inclusivement, et R' représente un atome d'hydrogène ou un radical alkyle contenant 1 à 4 atomes de carbone éventuellement substitué par un radical phényle, ou un radical de formule générale : 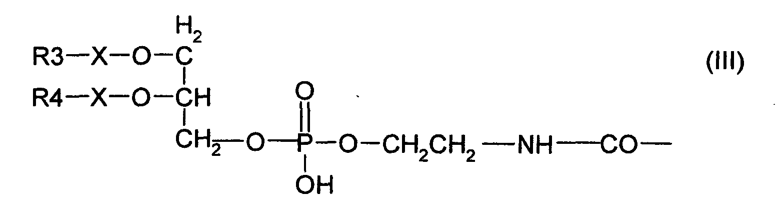 dans lequel X représente un groupement méthylène (-CH₂-) ou un groupement carbonyle (-CO-), et R₃ et R₄, identiques ou différents, représentent chacun un radical aliphatique saturé C_{p'}H_{2p'+2} ou insaturé C_{p'}H_{2p'} ou C_{p'}H_{2p'-2}, p' étant un nombre entier compris entre 11 et 21 inclusivement, étant entendu que :
- quelles que soient les valeurs de m et n, un seul des symboles R représente un radical de formule générale (II) ou (III).

3. Composition selon la revendication 1, **caractérisée en ce que** la lipopolyamine est choisie parmi le DOGS et le DPPES.

4. Composition selon la revendication 1, **caractérisée en ce que** l'acide nucléique est un acide désoxyribonucléique ou ribonucléique.

5. Composition selon la revendication 4, **caractérisée en ce que** l'acide nucléique est antisens.

6. Composition selon la revendication 4, **caractérisée en ce que** l'acide nucléique comporte un gène thérapeutique.

7. Composition selon l'une des revendications précédentes, **caractérisée en ce que** le rapport charges positives de la lipopolyamine sur charges négatives de l'acide nucléique est compris entre 0,1 et 1,9.

8. Composition selon la revendication 7, **caractérisée en ce que** le rapport charges positives de la lipopolyamine sur charges négatives de l'acide nucléique est compris entre 0,5 et 1,5.

9. Composition comprenant un acide nucléique, une lipopolyamine dans laquelle la région polyamine est la spermine, **caractérisée en ce qu'**elle comporte en outre un lipide neutre dioléoylphosphatidyléthanolamine (DOPE), et que le rapport charges positives de la lipopolyamine sur charges négatives de l'acide nucléique est inférieur ou égal à 2.

10. Composition selon la revendication 9, **caractérisée en ce que** la lipopolyamine est définie selon l'une quelconque des revendications 2 à 3.

11. Composition selon l'une des revendications 9 à 10, **caractérisée en ce qu'**elle comprend de 0,1 à 20 équivalents de lipides neutres pour 1 équivalent de lipopolyamine, et plus préférentiellement, de 1 à 5.

12. Composition selon l'une quelconque des revendications 9 à 11, pour une utilisation comme médicament.

13. Composition selon l'une des revendications précédentes, **caractérisée en ce qu'**elle comprend un véhicule pharmaceutiquement acceptable pour une formulation injectable.

14. Composition selon l'une quelconque des revendications précédentes, **caractérisée en ce qu'**elle comprend un véhicule pharmaceutiquement acceptable pour une application sur la peau et/ou les muqueuses.

## Patentansprüche

1. Zusammensetzung, die eine Nucleinsäure und ein Lipopolyamin umfasst, wobei es sich bei dem Polyaminbereich um Spermin handelt, **dadurch gekennzeichnet, dass** das Verhältnis von positiven Ladungen des Lipopolyamins zu negativen Ladungen der Nucleinsäure kleiner oder gleich 2 ist, zur Verwendung als Medikament.

2. Zusammensetzung gemäß Anspruch 1, **dadurch gekennzeichnet, dass** das Lipopolyamin der allgemeinen Formel 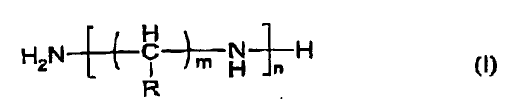 entspricht, wobei:
- n und m ganze Zahlen sind, die so gewählt sind, dass eine Spermingruppe entsteht;
- R Folgendes darstellt: ein Wasserstoffatom oder einen Rest der allgemeinen Formel 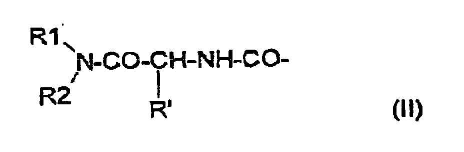 wobei R₁ und R₂ gleich oder verschieden sind und jeweils einen gesättigten aliphatischen CₚH₂ₚ₊₂-Rest oder ungesättigten aliphatischen CₚH₂ₚ- oder CₚH₂ₚ₋₂-Rest darstellen, wobei p eine ganze Zahl zwischen 12 und 22 einschließlich ist, und R' ein Wasserstoffatom oder einen Alkylrest mit 1 bis 4 Kohlenstoffatomen, der gegebenenfalls mit einem Phenylrest substituiert ist, darstellt;
oder einen Rest der allgemeinen Formel 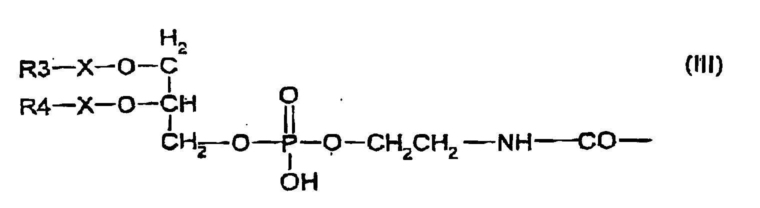 wobei X eine Methylengruppe (-CH₂-) oder eine Carbonylgruppe (-CO-) darstellt und R₃ und R₄ gleich oder verschieden sind und jeweils einen gesättigten aliphatischen C_{p'}H_{2p'+2}-Rest oder ungesättigten aliphatischen C_{p'}H_{2p'}- oder C_{p'}H_{2p'-2}-Rest darstellen, wobei p' eine ganze Zahl zwischen 11 und 21 einschließlich ist, mit der Maßgabe, dass;
- unabhängig von den Werten von m und n nur ein einziges der Symbole R einen Rest der allgemeinen Formel (II) oder (III) darstellt.

3. Zusammensetzung gemäß Anspruch 1, **dadurch gekennzeichnet, dass** das Lipopolyamin aus DOGS und DPPES ausgewählt ist.

4. Zusammensetzung gemäß Anspruch 1, **dadurch gekennzeichnet, dass** die Nucleinsäure eine Desoxyribo- (DNA) oder Ribonucleinsäure (RNA) ist.

5. Zusammensetzung gemäß Anspruch 4, **dadurch gekennzeichnet, dass** die Nucleinsäure eine Antisense-Nucleinsäure ist.

6. Zusammensetzung gemäß Anspruch 4, **dadurch gekennzeichnet, dass** die Nucleinsäure ein therapeutisches Gen umfasst.

7. Zusammensetzung gemäß einem der vorstehenden Ansprüche, **dadurch gekennzeichnet, dass** das Verhältnis von positiven Ladungen des Lipopolyamins zu negativen Ladungen der Nucleinsäure zwischen 0,1 und 1,9 liegt.

8. Zusammensetzung gemäß Anspruch 7, **dadurch gekennzeichnet, dass** das Verhältnis von positiven Ladungen des Lipopolyamins zu negativen Ladungen der Nucleinsäure zwischen 0,5 und 1,5 liegt.

9. Zusammensetzung, die eine Nucleinsäure und ein Lipopolyamin, bei dessen Polyaminbereich es sich um Spermin handelt, umfasst, **dadurch gekennzeichnet, dass** sie außerdem ein neutrales Dioleylphosphatidylethanolamin-Lipid (DOPE) umfasst und dass das Verhältnis von positiven Ladungen des Lipopolyamins zu negativen Ladungen der Nucleinsäure kleiner oder gleich 2 ist.

10. Zusammensetzung gemäß Anspruch 9, **dadurch gekennzeichnet, dass** das Lipopolyamin gemäß einem der Ansprüche 2 bis 3 definiert ist.

11. Zusammensetzung gemäß einem der Ansprüche 9 bis 10, **dadurch gekennzeichnet, dass** sie 0,1 bis 20 und vorzugsweise 1 bis 5 Äquivalente neutrale Lipide auf 1 Äquivalent Lipopolyamin umfasst.

12. Zusammensetzung gemäß einem der Ansprüche 9 bis 11 zur Verwendung als Medikament.

13. Zusammensetzung gemäß einem der vorstehenden Ansprüche, **dadurch gekennzeichnet, dass** sie einen pharmazeutisch annehmbaren Träger für eine injizierbare Zubereitung umfasst.

14. Zusammensetzung gemäß einem der vorstehenden Ansprüche, **dadurch gekennzeichnet, dass** sie einen pharmazeutisch annehmbaren Träger für eine Applikation auf die Haut und/oder die Schleimhäute umfasst.

## Claims

1. composition comprising a nucleic acid and a lipvpolyamine, in which the polyamine region is spermine **characterized in that** the positive charges of the lipopolyamine to negative charges of the nucleic acid ratio is less than or equal to 2, for a use as a medicament.

2. Composition according to claim 1, **characterized in that** the lipopolyamine corresponds to the general formula: 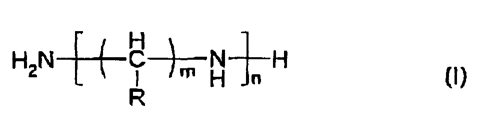
- n and m are integers chosen so as to form a spermine group,
- R represents a hydrogen atom or a radical of general formula: 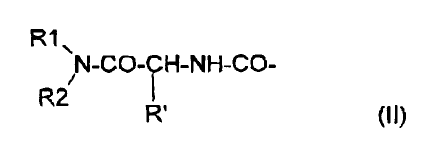 in which R₁ and R₂, identical or different, each represent a saturated CₚH₂ₚ₊₂ or unsaturated CₚH₂ₚ or CₚH₂ₚ₋₂ aliphatic radical, p being an integer comprised between 12 and 22 inclusively, and R' represents a hydrogen atom or an alkyl radical containing 1 to 4 carbon atoms optionally substituted by a phenyl radical, or a radical of the general formula: 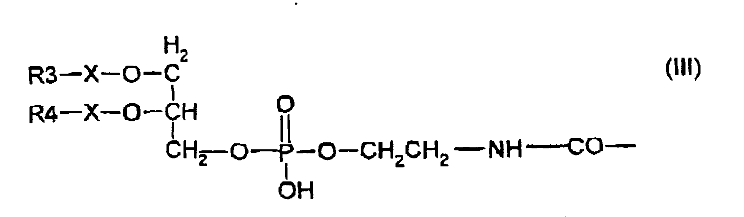 in which X represents a methylene group (-CH₂-) or a carbonyl group (-CO-), and R₃ and R₄, identical or different, each represent a saturated C_{p'}H_{2p'+2} or unsaturated C_{p'}H_{2p'} or C_{p'}H_{2p'-2} aliphatic radical, p' being an integer comprised between 11 and 21 inclusively, it being understood that:
- no matter what the values of m and n, only one of the symbols R represents a radical of general formula (II) or (III).

3. Composition according to claim 1, **characterized in that** the lipopolyamine is chosen from DOGS and DPPES.

4. Composition according to claim 1, **characterized in that** the nucleic acid is a deoxyribonucleic or ribonucleic acid.

5. Composition according to claim 4, **characterized in that** the nucleic acid is antisense.

6. Composition according to claim 4, **characterized in that** the nucleic acid comprises a therapeutic gene.

7. Composition according to one of the previous claims, **characterized in that** the positive charges of the lipopolyamine to negative charges of the nucleic acid ratio is comprised between 0.1 and 1.9.

8. Composition according to claim 7, **characterized in that** the positive charges of the lipopolyamine to negative charges of the nucleic acid ratio is comprised between 0.5 and 1.5.

9. Composition comprising a nucleic acid, a lipopolyamine in which the polyamine region is spermine, **characterized in that** it also comprises a neutral lipid dioleoylphosphatidylethanolamine (DOPE), and **in that** the positive charges of the lipopolyamine to negative charges of the nucleic acid ratio is less than or equal to 2.

10. Composition according to claim 9, **characterized in that** the lipopolyamine is defined according to any one of claims 2 to 3.

11. Composition according to one of claims 9 to 10, **characterized in that** it comprises from 0.1 to 20 equivalents of neutral lipids to 1 equivalent of lipopolyamine, and more preferably from 1 to 5.

12. Composition according to any one of claims 9 to 11, for a use as a medicament.

13. Composition according to one of the previous claims, **characterized in that** it comprises a vehicle which is pharmaceutically acceptable for an injectable formulation.

14. Composition according to any one of the previous claims, **characterized in that** it comprises a vehicle which is pharmaceutically acceptable for an application on the skin and/or mucous membranes.
